# EUROPÄISCHE PATENTSCHRIFT

(11) **EP 3 178 438 B1**
(45) Veröffentlichungstag und Bekanntmachung des Hinweises auf die Patenterteilung: **11.04.2018**
(21) Anmeldenummer: 16199724.2
(22) Anmeldetag: 21.11.2016
(51) Int. Cl.: A61B 46/10, A61B 90/20

(54) **SCHUTZADAPTER FÜR EIN OPERATIONSMIKROSKOP**
PROTECTING ADAPTER FOR AN OPERATION MICROSCOPE
ADAPTATEUR DE PROTECTION POUR UN MICROSCOPE D'OPÉRATION

(30) Priorität: 11.12.2015 DE 102015225009
(43) Veröffentlichungstag der Anmeldung: 14.06.2017
(73) Patentinhaber: Carl Zeiss Meditec AG, 07745 Jena (DE)
(72) Erfinder: König, Frank, 73431 Aalen (DE); Lindner, Daniel, 89551 Königsbronn (DE)
(74) Vertreter: Carl Zeiss AG - Patentabteilung

(56) Entgegenhaltungen:
- DE-A1-102007 043 997
- US-A- 3 528 720
- US-A- 5 467 223
- US-A- 5 682 264

## Beschreibung

Die Erfindung betrifft einen Schutzglasadapter für ein Operationsmikroskop. anzubringen. Das Schutzglasmodul umfasst einen Schutzglasadapter und ein Objektivschutzglas. Der Schutzglasadapter ist mechanisch mit dem Operationsmikroskop verbunden. An dem Schutzglasadapter ist das Objektivschutzglas angeordnet, sodass durch das Hauptobjektiv und das Objektivschutzglas hindurch ein Operationssitus beobachtbar ist. Zusätzlich kann der Schutzglasadapter mit einer sterilen Abdeckfolie verbunden sein, um das gesamte Operationsmikroskop während einer Operation vor Verschmutzungen zu schützen.

Schutzglasfassung gewähren seitliche Führungslaschen Führungskontakt mit dem Operationsmikroskop. Danach greifen Rastbolzen in Aufnahmebohrungen und eine Nase in die für sie vorgesehene Ausnehmungen ein. Die exakte Positionierung der Schutzglasfassung Druckstift entgegen der Federkraft bewegt werden, vergrößert sich die Kraft, die aufgebracht werden muss, um den Schutzglasadapter an dem Operationsmikroskop anbringen zu können. Diese Kräfte können bewirken, dass das Operationsmikroskop gehalten werden muss, damit es seine Position während der Adaption des Schutzglasadapters nicht verändert.

Weiterhin ist aus der US3,528,720 ein Schutzglasadapter für ein Operationsmikroskop bekannt mit einer ersten Anlagefläche und einer kegelstumpfförmigen Führungsfläche gemäß des Oberbegriffs von Anspruch 1. Operationsmikroskop anbringbar ist, ohne dass dabei das Operationsmikroskop gehalten werden muss.

Die Aufgabe wird durch eine Vorrichtung mit den Merkmalen des unabhängigen Anspruchs 1 gelöst. Vorteilhafte Weiterbildungen der Erfindung sind in den Unteransprüchen beschrieben.

Erfindungsgemäß weist der der Schutzglasadapter für ein Operationsmikroskop eine erste Anlagefläche auf, wobei die erste Anlagefläche derart ausgebildet ist, dass diese mit einer korrespondierenden zweiten Anlagefläche des Operationsmikroskops in Kontakt bringbar ist. Der Schutzglasadapter weist eine Führungsöffnung zur Aufnahme eines Hauptobjektivs des Operationsmikroskops auf, wobei die Führungsöffnung als Kegelstumpf mit einer ersten Zentralachse ausgebildet ist, wobei der größere Durchmesser des Kegelstumpfes zu der ersten Anlagefläche hin ausgebildet ist. Der Schutzglasadapter weist mindestens ein Positionierungselement auf, wobei das mindestens eine Positionierungselement starr ausgebildet ist und an der ersten Anlagefläche oder an der Oberfläche der Führungsöffnung angeordnet ist, wobei das Positionierungselement zur formschlüssigen Aufnahme in einem Gegenelement an dem Operationsmikroskop geeignet ist. Der Schutzglasadapter weist mindestens ein Halteelement auf, wobei durch das Halteelement eine Haltekraft parallel zu der ersten Zentralachse zwischen dem Schutzglasadapter und dem Operationsmikroskop bewirkbar ist, wobei die Haltekraft durch eine Magnetkraft gebildet ist.

Der erfindungsgemäße Schutzglasadapter kann sehr leicht an einem Operationsmikroskop angebracht werden. Ein Operationsmikroskop weist ein Hauptobjektiv mit einer optischen Achse auf.

Die Führungsöffnung des Schutzglasadapters ist als Kegelstumpf mit einer ersten Zentralachse ausgebildet. Damit kann der der Schutzglasadapter sehr einfach über das Hauptobjektiv gestülpt werden. Durch den großen Durchmesser ist zunächst zwischen dem Hauptobjektiv und dem Schutzglasadapter ein sehr großes Spiel ausgebildet. Diese Position des Schutzglasadapters kann somit vom Benutzer, ohne hinzuschauen, leicht erfühlt werden. Beim Bewegen des Schutzglasadapters in Richtung der optischen Achse zu der zweiten Anlagefläche an dem Operationsmikroskop wird der Schutzglasadapter durch die Kegelform der Führungsöffnung zentriert und geführt.

Dabei findet praktisch kaum eine Reibung zwischen dem Schutzglasadapter und dem Hauptobjektiv statt. Die Reibungsfläche beschränkt sich auf eine punkt- oder dünne linienförmige Berührfläche. Der Schutzglasadapter kann sehr leicht und ohne dabei eine nennenswerte Kraft auf das Operationsmikroskop auszuüben, passgenau zu der zweiten Anlagefläche an das Operationsmikroskop geführt werden.

Das an der ersten Anlagefläche angeordnete Positionierungselement bewirkt zudem, das die erste Anlagefläche des Schutzglasadapters noch nicht großflächig mit der korrespondierenden zweiten Anlagefläche des Operationsmikroskops in Kontakt kommt. Vielmehr entsteht lediglich eine sehr geringe Reibung zwischen der sehr kleinen Kontaktfläche des Positionierungselements und der zweiten Anlagefläche des Operationsmikroskops. Die Ausgestaltung der Führungsfläche als Kegelstumpf bewirkt zudem, dass zwischen dem Schutzglasadapter und dem Hauptobjektiv noch ein gewisses Spiel ausgebildet ist, sodass die Reibung zwischen Schutzglasadapter und Hauptobjektiv sich immer noch auf eine punkt- oder linienförmige Berührung beschränkt. Durch leichte links/rechts-Bewegungen des Schutzglasadapters gleitet das Positionierungselement in ein korrespondierendes Gegenelement des Operationsmikroskops. Dabei verringert sich der Abstand zwischen den beiden Kontaktflächen des Schutzglasadapters und des Operationsmikroskops. An dieser Position ist die magnetische Haltekraft sehr groß und der Schutzglasadapter wird ausschließlich durch eine magnetische Haltekraft an dieser Position gehalten. Erst in dieser Position ist das Spiel zwischen der als Kegelstumpf ausgebildeten Oberfläche der Führungsöffnung und des Hauptobjektives minimal, sodass der Schutzglasadapter passgenau und in einer korrekten Position am Hauptobjektiv angeordnet ist.

Die leichte Adaption des Schutzglasadapters an ein Operationsmikroskop soll durch folgende kinematische Betrachtung nochmals verdeutlicht werden.

Die optische Achse des Hauptobjektivs definiert eine Z-Achse. Senkrecht zu dieser Z-Achse ist eine X-Y-Ebene ausgebildet. Wenn der Schutzglasadapter über das Hauptobjektiv geführt ist und die erste Anlagefläche nicht mit der korrespondierenden zweiten Anlagefläche in Kontakt ist, ist der Schutzglasadapter gemäß des zwischen Führungsöffnung und Hauptobjektiv ausgebildeten Spiels in der X-Y-Ebene verschiebbar und bezüglich der X-Y-Ebene verkippbar. Zudem ist der Schutzglasadapter um die Zentralachse der Führungsöffnung, die im Wesentlichen parallel zur optischen Achse geführt ist, frei drehbar.

Erst durch den Formschluss der ersten Anlagefläche des Schutzglasadapters mit der korrespondierenden zweiten Anlagefläche des Operationsmikroskops ist der Schutzglasadapter in einer Ebene parallel zur ersten Anlagefläche ausgerichtet. Die Führungsöffnung bewirkt, ausgenommen von dem verbleibenden geringen Restspiel zwischen Führungsöffnung und dem Hauptobjektiv in dieser Lage, dass der Schutzglasadapter in der X-Y-Ebene fixiert ist. Das Positionierungselement ist in dieser Position formschlüssigen in dem Gegenelement an dem Operationsmikroskop aufgenommen und fixiert den Schutzglasadapter in Rotationsrichtung bezüglich der Zentralachse, bzw. der Z-Achse. Die Position des Schutzglasadapters in Bezug auf das Operationsmikroskop ist damit bezüglich aller Freiheitsgrade eindeutig definiert. Nur in dieser Position bewirkt das Halteelement eine magnetische Haltekraft zwischen dem Schutzglasadapter und dem Operationsmikroskop.

Die korrekte Position des Schutzglasadapters kann somit sehr schnell und einfach erfühlt werden. Der Schutzglasadapter kann mit einer Hand und ohne dass dabei eine nennenswerte Reibung oder Kraft auf das Operationsmikroskop ausgeübt wird, sehr schnell und einfach am Hauptobjektiv befestigt werden. Deshalb ist es auch nicht notwendig das Operationsmikroskop mit der anderen Hand zu halten. Zudem ist diese Verbindung jederzeit sehr leicht und einfach wieder lösbar.

Alternativ ist eine Anordnung des mindestens einen Positionierungselements an der Oberfläche der Führungsöffnung vorstellbar. Bei dieser Anordnung kann das mindestens eine korrespondierende Gegenelement am Hauptobjektiv des Operationsmikroskops ausgebildet sein.

In einer Ausgestaltung der Erfindung ist das mindestens eine Positionierungselement halbkugelförmig ausgebildet ist.

Ein halbkugelförmiges Positionierungselement kann auch bei einem leichten Verkippen des Schutzglasadapters sehr leicht in ein korrespondierendes Gegenelement formschlüssig eingeführt werden. Wenn das Positionierungselement an einer Position außerhalb des Gegenelementes mit der komplementären Anlagefläche des Operationsmikroskops in Kontakt ist, ist durch die halbrunde Form nur eine kleine punktförmige Kontaktfläche ausgebildet. Ein weiterer Vorteil ist, dass der Schutzglasadapter durch die halbkugelförmige Oberfläche bei seitlichem Drehen um die Zentralachse leichter aus dem Gegenelement herausgedreht werden kann.

In einer Ausgestaltung der Erfindung sind zwei Positionierungselemente an der ersten Anlagefläche oder an der Oberfläche der Führungsöffnung angeordnet.

Durch zwei Positionierungselemente ist eine eindeutige Position des Schutzglasadapters an dem Operationsmikroskop auch bei großem Spiel zwischen der Führungsöffnung und dem Hauptobjektiv definiert. Zudem kann eine Verkippung der Zentralachse des Schutzglasadapters leichter erfühlt werden, wenn die zwei Positionierungselemente mit der komplementären zweiten Anlagefläche des Operationsmikroskops in Kontakt sind.

In einer Ausgestaltung der Erfindung sind drei Positionierungselemente an der ersten Anlagefläche oder an der Oberfläche der Führungsöffnung angeordnet.

Wenn das Positionierungselement an einer Position außerhalb der Gegenelemente mit der komplementären zweiten Anlagefläche des Operationsmikroskops in Kontakt ist, ist diese Kontaktfläche durch drei Kontaktpunkte gebildet. Damit kann ein Verkippen der Zentralachse des Schutzglasadapters in dieser Kontaktposition zuverlässig vermieden werden. Bei einer Anordnung der drei Positionierungselemente an der Oberfläche der Führungsöffnung kann die Außenfläche des Hauptobjektivs die Kontaktfläche des Operationsmikroskops bilden.

In einer Ausgestaltung der Erfindung sind mindestens zwei Halteelemente an dem Schutzglasadapter angeordnet.

Vorteilhaft ist durch mindestens zwei Halteelemente eine bessere Haltewirkung erreichbar. Die beiden Halteelemente sind vorteilhaft als magnetische Halteelemente ausgebildet.

In einer Ausgestaltung der Erfindung weist die Führungsöffnung einen Kegelwinkel zwischen 10° und 45°, bevorzugt zwischen 10° und 20°, auf.

Durch die Kegelform der Führungsöffnung ist ein relativ großes Spiel und damit praktisch gar keine oder nur minimale Reibung zwischen Schutzglasadapter und dem Hauptobjektiv des Operationsmikroskops erreichbar. Dieses Spiel wird erst im Moment des Formschlusses der ersten Anlagefläche des Schutzglasadapters mit der zweiten Anlagefläche des Operationsmikroskops minimal.

In einer Ausgestaltung der Erfindung umfasst der Schutzglasadapter ein Objektivschutzglas.

Damit kann ein vorteilhaft ein komplettes Schutzglasmodul bereitgestellt werden. Die gleichen Vorteile wie für den Schutzglasadapter können für das Schutzglasmodul, umfassend einen Schutzglasadapter ein Objektivschutzglas, erreicht werden.

In einer Ausgestaltung der Erfindung ist das Objektivschutzglas trennbar mit dem Schutzglasadapter verbunden und in einer Aufnahmevorrichtung aufgenommen.

Damit kann vorteilhaft das Objektivschutzglas, beispielsweise bei Verschmutzung, ausgetauscht werden, ohne dass der Schutzglasadapter von einem Operationsmikroskop entfernt werden muss.

In einer Ausgestaltung der Erfindung ist an dem Schutzglasadapter eine Mikroskopabdeckung angeordnet.

Durch die Mikroskopabdeckung, eine flexible Abdeckfolie, ist das Operationsmikroskop abdeckbar. Vorteilhaft kann mit einer Hand der Schutzglasadapter am Operationsmikroskop angebracht werden während mit der andere Hand die Mikroskopabdeckung gehalten werden kann. Die Handhabung eines Gesamtsystems aus Schutzglasadapter und Mikroskopabdeckung ist somit sehr einfach. Zudem kann durch die Führungsöffnung und/oder die Anlagefläche des Schutzglasadapters eine besonders gute Abdichtung zwischen Mikroskopabdeckung und Operationsmikroskop erreicht werden.

In einer Ausgestaltung der Erfindung ist die Mikroskopabdeckung an der ersten Anlagefläche angeordnet.

Damit ist die Herstellung dieser Verbindung sehr einfach möglich. Zudem ist eine besonders gute Abdichtung zwischen Mikroskopabdeckung und Operationsmikroskop im Bereich des Schutzglasadapters erreichbar.

Ein Operationsmikroskop umfasst vorteilhaft einen wie vorstehend beschriebene Schutzglasadapter. Das Operationsmikroskop weist eine zweite Anlagefläche auf, die mit der ersten Anlagefläche des Schutzglasadapters in Kontakt bringbar ist. Das Operationsmikroskop weist ein Hauptobjektiv mit einer äußeren Umfangsfläche auf, wobei die äußere Umfangsfläche als Kegelstumpf ausgebildet ist, wobei der größere Durchmesser des Kegelstumpfes zu der zweiten Anlagefläche hin ausgebildet ist. Das Operationsmikroskop weist mindestens ein Gegenelement auf, das in Position und Form mit dem mindestens einen Positionierungselement in Formschluss bringbar ist. Das Operationsmikroskop weist Mindestens ein Gegen-Halteelement auf, das derart ausgebildet ist, dass eine magnetische Haltekraft zwischen dem mindestens einen Halteelement und dem mindestens einen Gegen-Halteelement bewirkbar ist.

Der Schutzglasadapter kann sehr schnell, und ohne das Operationsmikroskop halten zu müssen, über das Hauptobjektiv zentriert und geführt an dem Operationsmikroskop angebracht werden. Damit sind die oben beschriebenen Vorteile erreichbar. Durch die kompakte Anordnung des Schutzglasadapters an dem Operationsmikroskop kann dieser vorteilhaft an einem Operationsmikroskop angebracht werden, sodass der Arbeitsraum zwischen Operationsmikroskop und Operationssitus nur in geringem Maße eingeschränkt wird.

In einer Ausgestaltung der Erfindung umfasst ein Operationsmikroskop einen Schutzglasadapter, wobei die Führungsöffnung des Schutzglasadapters über das Hauptobjektiv geführt ist. Der Schutzglasadapter ist rotatorisch um die Zentralachse beweglich ist, wenn das mindestens eine Positionierungselement in Kontakt mit der zweiten Anlagefläche steht, sodass die erste Anlagefläche durch das Positionierungselement in einem Abstand zu der zweiten Anlagefläche des Operationsmikroskops angeordnet ist, wenn das Positionierungselement nicht in Formschluss mit dem Gegenelement positioniert ist. Die Haltekraft zwischen dem mindestens einen Halteelement und dem mindestens ein Gegen-Halteelement ist ausschließlich an einer ersten Position ausgebildet, an der das Positionierungselement an einer vorbestimmten Position in Bezug auf das Operationsmikroskop positioniert ist und das Positionierungselement in dem Gegenelement in Bezug auf das Operationsmikroskop formschlüssig angeordnet ist, sodass die erste Anlagefläche ausschließlich an der ersten Position mit der korrespondierenden zweiten Anlagefläche des Operationsmikroskops in Kontakt ist.

Damit ist der Schutzglasadapter vorteilhaft sehr leicht und einfach an dem Operationsmikroskop anbringbar.

In einer Ausgestaltung der Erfindung weist die Aufnahmeeinrichtung des Schutzglasadapters mindestens eine lösbare Rastvorrichtung für das Objektivschutzglas auf.

Die lösbare Rastvorrichtung bewirkt, dass das Objektivschutzglas während des Einsatzes sicher in der Aufnahmevorrichtung des Schutzglasadapters gehalten ist und dennoch trennbar mit dem Schutzglasadapter verbunden ist.

In einer Ausgestaltung der Erfindung ist der Schutzglasadapters mit dem Objektivschutzglas einteilig ausgebildet.

Damit kann das Schutzglasmodul kostengünstiger hergestellt werden, da eine spezielle Aufnahmevorrichtung am Schutzglasadapter für das Objektivschutzglas entfällt. Damit kann eine Gewichtsreduktion für das Schutzglasmodul erreicht werden.

In einer Ausgestaltung der Erfindung ist der Schutzglasadapter als Ring ausgeführt.

Damit ist eine kostengünstigere Herstellung der Werkzeuge möglich, die zur Produktion eines Schutzglasadapters aus Kunststoff verwendet werden.

Weitere Vorteile und Merkmale der Erfindung werden mit Bezug auf die nachfolgenden Zeichnungen erklärt, in welchen zeigen:
- Figur 1: eine schematische Darstellung eines Operationsmikroskop mit einem Schutzglasmodul in einer Schnittdarstellung;
- Figur 2: eine Darstellung einer ersten Ausführungsform eines erfindungsgemäßen Schutzglasadapters mit einem Schutzglas;
- Figur 3: eine schematische Schnittdarstellung eines Schutzglasadapters, der über ein Hauptobjektiv geführt ist;
- Figur 4: eine schematische Darstellung eines Schutzglasadapter gemäß Figur 3, der an einem Operationsmikroskop angeordnet ist.

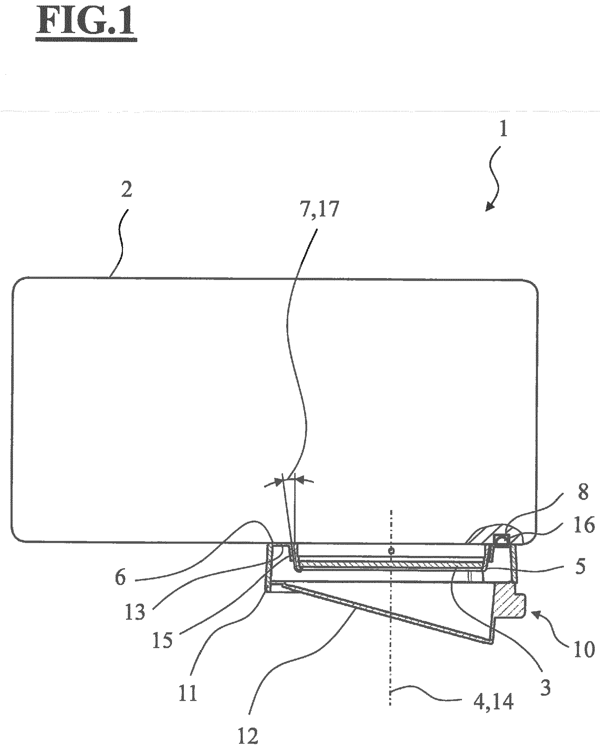

Figur 1 zeigt eine schematische Darstellung eines Operationsmikroskops mit einem Schutzglasmodul.

Ein Operationsmikroskop-System 1 umfasst ein Operationsmikroskop 2 mit einem Schutzglasmodul 10. Das Operationsmikroskop 2 weist ein Hauptobjektiv 3 mit einer optischen Achse 4 auf. Die Außenform des Hauptobjektivs 3 ist als Kegelstumpf mit einem zweiten Kegelwinkel 7 und einer äußeren Mantelfläche 5 ausgebildet. Der größere Durchmesser des Kegelstumpfes zeigt zum Operationsmikroskop 2.

An der Unterseite des Operationsmikroskops 2 ist das Schutzglasmodul 10 angeordnet ist. Das Schutzglasmodul 10 umfasst einen Schutzglasadapter 11 und ein Objektivschutzglas 12. Dabei ist eine erste Anlagefläche 13 des Schutzglasmoduls 10 mit einer korrespondierenden zweiten Anlagefläche 6 des Operationsmikroskops in Kontakt. Das Schutzglasmodul 10 ist trennbar an dem Operationsmikroskop 2 angeordnet. Das Objektivschutzglas 12 ist trennbar mit dem Schutzglasadapter 11 verbunden.

Der Schutzglasadapter 11 weist eine Führungsöffnung 15 zur Aufnahme des Hauptobjektivs 3 auf. Die Führungsöffnung 15 ist als Kegelstumpf mit einem ersten Kegelwinkel 17 und einer ersten Zentralachse 14 ausgebildet. Der größere Durchmesser des Kegelstumpfes ist zu der ersten Anlagefläche 13 hin ausgebildet. Der erste Kegelwinkel 17 der Führungsöffnung 15 hat den gleichen Betrag wie der zweite Kegelwinkel 7 der äußeren Mantelfläche 5 des Hauptobjektivs 3. Die Zentralachse 14 fällt mit der optischen Achse 4 des Hauptobjektivs 3 zusammen. Das Objektivschutzglas 12 ist in einem Winkel größer oder kleiner als 90° zu der der optischen Achse 14 angeordnet, um Reflexionen in der Mikroskopoptik, die das Hauptobjektiv 3 umfasst, zu vermeiden. Die definierte Neigung des Objektivschutzglases ermöglicht eine reflexfreie Sicht auf einen Operationssitus.

Auf der ersten Anlagefläche 13 ist ein erstes Positionierungselement 16 angeordnet. Das erste Positionierungselement 16 ist starr als Halbkugel ausgebildet. Die zweite Anlagefläche 6 des Operationsmikroskops 2 weist ein erstes Gegenelement 8 auf, das als Aussparung oder Bohrung ausgebildet ist. Dabei greift das erste Positionierungselement 16 formschlüssig in das erste Gegenelement 8 ein, wenn die erste Anlagefläche 13 mit der zweiten Anlagefläche 6 in Kontakt ist, sodass der Schutzglasadapter 11 in Bezug auf das Hauptobjektiv 3 exakt positioniert ist. Das formschlüssige Eingreifen ist derart ausgebildet, dass das erste Positionierungselement 16 mit einer Kontaktlinie formschlüssig in dem Gegenelement 8 positioniert ist. Das erste Positionierungselement 16 kann als Halbkugel ausgebildet sein und das Gegenelement 8 als Bohrung. Die Kontaktlinie kann eine Kreislinie auf der Halbkugel sein.

In dieser Position wird das Schutzglasadapter 10 durch eine Haltekraft an dem Operationsmikroskop gehalten. Die Haltekraft ist ausschließlich durch eine Magnetkraft gebildet. Dazu sind an dem Schutzglasadapter 10 nicht dargestellte Halteelemente angeordnet, die mit korrespondierenden, nicht dargestellten, magnetischen Gegen-Halteelementen am Operationsmikroskop eine Magnetkraft ausbilden können. Die Haltekraft ist parallel zu der ersten Zentralachse 14 zwischen dem Schutzglasadapter 10 und dem Operationsmikroskop 2 ausgebildet. Die Halteelemente oder Gegen-Halteelemente können Dauermagnete in Kombination mit anderen Magneten oder ferromagnetischen Metalle, beispielsweise Eisen, umfassen. Vorzugsweise weisen die Halteelemente, die an dem Schutzglasadapter angeordnet sind aus Kostengründen Eisen auf. Die Magnete an dem Operationsmikroskop 2 können auch als Elektromagnete ausgebildet sein.

Alternativ ist vorstellbar, dass die Haltekraft durch eine Reibungskraft und/oder Klebekraft oder eine Kombination aus Reibungskraft und/oder Klebekraft und Magnetkraft gebildet ist.

Der Kegelwinkel der Führungsöffnung 15 kann in einem Bereich zwischen 1° und 45° ausgebildet sein, bevorzugte Werte sind beispielsweise 10° oder 15°. Es ist sogar vorstellbar, dass die Führungsöffnung 15 in einer konkaven oder konvexen Form ausgebildet ist. Es ist auch vorstellbar, dass die Außenform des Hauptobjektivs 3 eine konkave oder konvexe Außenform bildet.

Der Schutzglasadapter 11 und/oder das Objektivschutzglas 12 können aus Kunststoff bestehen. Es ist auch vorstellbar, dass der Schutzglasadapter 11 zusammen mit dem Objektivschutzglas 12 einteilig ausgebildet ist. Das Objektivschutzglas 12 ist transparent.

Es ist auch vorstellbar, dass das erste Positionierungselement 16 und das erste Gegenelement 8 jeweils invertiert ausgebildet sind, d. h. das erste Positionierungselement 16 ist als eine Aussparung oder ein Loch gebildet und das erste Gegenelement ist als eine auf der zweiten Anlagefläche 6 angeordnete Halbkugel gebildet, die formschlüssig in die Aussparung oder das Loch eingreifen kann.

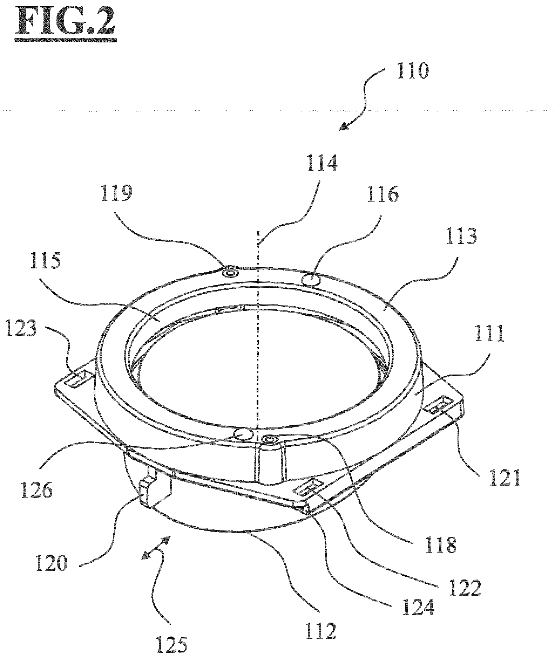

Figur 2 zeigt eine Darstellung einer ersten Ausführungsform eines erfindungsgemäßen Schutzglasadapters 111 mit einem Objektivschutzglas 112.

Ein Schutzglasmodul 110 weist die gleichen Komponenten auf wie das Schutzglasmodul 10 gemäß Figur 1. Die Bezugszeichen sind in Bezug auf das Schutzglasmodul 10 jeweils um 100 erhöht.

Das Schutzglasmodul 110 umfasst den Schutzglasadapters 111 und das Objektivschutzglas 112. Das Objektivschutzglas 112 ist transparent.

Das Schutzglasmodul umfasst eine erste Anlagefläche 113, die als Planfläche ausgeführt ist. Eine Führungsöffnung 115 ist als Kegelstumpf mit einer Zentralachse 114 ausgebildet. Der größere Durchmesser des Kegelstumpfes ist zu der ersten Anlagefläche 113 hin ausgebildet.

Auf der ersten Anlagefläche 113 sind ein erstes Positionierungselement 116 und ein zweites Positionierungselement 126 angeordnet. Das erste Positionierungselement 116 und das zweite Positionierungselement 126 sind jeweils starr als Halbkugel ausgebildet. Der Mittelpunkt der beiden Halbkugeln ist dabei jeweils unterhalb der Anlagefläche 113 definiert, so dass die Höhe der beiden Halbkugeln kleiner ist als der jeweilige Radius. Dies hat die Wirkung, dass am Übergang zwischen der Anlagefläche 113 und dem ersten Positionierungselement 116, bzw. dem zweiten Positionierungselement 126 jeweils ein Winkel ausgebildet ist, der größer ist als 90°. Dies erleichtert das Lösen des Schutzglasmoduls 110 von einem Operationsmikroskop, wenn das Schutzglasmodul 110 um die Zentralachse 114 gedreht wird. Die Mittelpunkte des ersten Positionierungselements 116 und des zweiten Positionierungselements 126 liegen dabei, bezogen auf die Zentralachse 114, nicht genau gegenüber, um zu vermeiden, dass der Schutzglasadapter an zwei unterschiedlichen Positionen bezüglich der Zentralachse 114 am Operationsmikroskop adaptierbar wäre.

An der ersten Anlagefläche 113 sind ein erstes Halteelement 118 und ein zweites Halteelement 119 angeordnet. Die Halteelemente 118, 119 können bündig an der ersten Anlagefläche 113 angeordnet sein, oder sich in einem geringen Abstand, beispielsweise 0.3 mm, unterhalb der ersten Anlagefläche 113 in den Schutzglasadapter eingebracht sein.
Das erste Halteelement 118 und das zweite Halteelement 119 bewirken, dass eine magnetische Haltekraft zwischen dem Schutzglasadapter 111 und dem Operationsmikroskop ausgebildet werden kann, die groß genug ist, den Schutzglasadapter 111 sicher am Operationsmikroskop zu halten. Dazu können die Halteelemente 118, 119 als Permanentmagnete ausgeführt sein, wobei die zugeordneten Halteelemente am Operationsmikroskop ebenfalls Permanentmagnete, elektrische Magnete oder ferromagnetische Metalle, z. B. Eisen, umfassen. Es ist auch vorstellbar, dass die Halteelemente 118, 119 Eisen umfassen und die zugeordneten Halteelemente am Operationsmikroskop Permanentmagnete, bevorzugt Neodym-Magnete oder elektrische Magnete bilden. Die Halteelemente 118, 119 können eingepresst, eingeklebt oder zusammen mit dem Schutzglasadapter vergossen werden. Die Halteelemente können von vorne, d. h. von der Anlagefläche 113 oder von der Rückseite in den Schutzglasadapter eingebracht werden.

Es ist auch vorstellbar, dass die Positionierungselemente 116, 126 als Halteelemente ausgebildet sind, die eine magnetische Haltekraft bewirken können.

Das Objektivschutzglas 112 ist in einer in einer Aufnahmevorrichtung, einer Führungsschiene 124, geführt und wird von vier Rastelementen gehalten. In Figur 2 ist ein erstes Rastelement 121, ein zweites Rastelement 122 und ein drittes Rastelement 123 dargestellt. Das Objektivschutzglas weist einen Griff 120 auf. Mit dem Griff 120 kann das Objektivschutzglas 120, wenn die Rastwirkung der Rastelemente 121, 122, 123 überwunden wird, in der Führungsschiene 124, verschoben werden, dargestellt durch den ersten Doppelpfeil 125. Auf diese Weise sind der Schutzglasadapter 111 und das Objektivschutzglas 112 trennbar miteinander verbunden. Es ist auch vorstellbar, dass die Rastelemente als seitlich angeordnete Rasthaken ausgebildet sind, die sich beispielsweise mittels eines Daumens und eines Fingers betätigen lassen.

Das Objektivschutzglas 112 besteht aus einem transparenten Material, bevorzugt Kunststoff. Die Unterseite des Objektivschutzglases ist bezüglich einer zu der Zentralachse 114 orthogonalen Ebene um einen Winkel, bevorzugt 15°, geneigt. Das Objektivschutzglas 112 ist damit derart ausgebildet, dass die Beobachtung eines Operationssitus nahezu uneingeschränkt möglich ist. Mögliche störende Reflexe werden durch die Schrägstellung des Objektivschutzglases weitgehend vermieden. Der Schutzglasadapter 111 und das Objektivschutzglas 112 sind sterilisierbar.

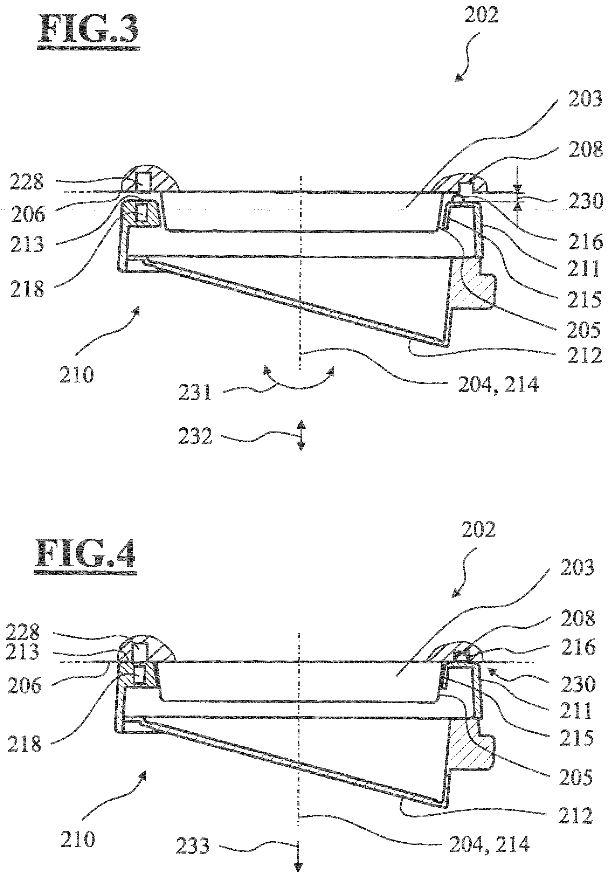

Figur 3 zeigt eine schematische Schnittdarstellung eines Schutzglasadapters, der über ein Hauptobjektiv geführt ist.

Ein Schutzglasmodul 210 gemäß Figur 3 weist die gleichen Komponenten auf wie das Schutzglasmodul 110 gemäß Figur 2. Die Bezugszeichen sind in Bezug auf das Schutzglasmodul 110 jeweils um 100 erhöht.

Das Schutzglasmodul 210 umfasst einen Schutzglasadapter 211 mit einem Objektivschutzglas 212. Der Schutzglasadapter 211 ist über ein Hauptobjektiv 203 geführt. Das Hauptobjektiv 203 hat eine optische Achse 204 und eine kegelförmige äußere Mantelfläche 205.

Eine erste Anlagefläche 213 des Schutzglasadapters hat einen Abstand 230 zu einer zweiten Anlagefläche 206 eines Operationsmikroskops 202. Zwischen der äußeren Mantelfläche 205 des Hauptobjektivs 203 und einer der kegelförmigen Führungsöffnung 215 des Schutzglasadapters mit einer Zentralachse 214 ist ein relativ großes Spiel ausgebildet. Der Schutzglasadapter 211 ist frei beweglich und damit sehr leicht um die Zentralachse 214 drehbar, dargestellt durch den zweiten Doppelpfeil 231, und entlang der Zentralachse 214 verschiebbar, dargestellt durch den dritten Doppelpfeil 232.

Ein erstes Positionierungselement 216 berührt die zweite Anlagefläche 206 in einem einzigen Punkt. Somit ist die Reibung zwischen Schutzglasadapter 211 und Operationsmikroskop minimal. An dem Schutzglasadapter kann ein nicht dargestelltes zweites oder drittes Positionierungselement angeordnet sein. Auch durch die Kontaktpunkte zwischen zweitem und drittem Positionierungselement ist die Reibung zwischen Schutzglasadapter und Operationsmikroskop minimal. Auch bei einer Verkippung des Schutzglasadapters 211 um die optische Achse 204, ist die Reibung auf die punkt- und/oder linienförmige Berührungen zwischen Schutzglasadapter 216 und Operationsmikroskop 202 beschränkt. In dieser Position ist keine Haltekraft zwischen dem ersten Halteelement 218, einem ferromagnetischen Eisenelement, und einem ersten Gegen-Halteelement 228, einem Dauermagneten, ausgebildet.

Durch eine leichte Drehbewegung des Schutzglasadapters 211 gleitet das erste Positionierungselement 216 in das korrespondierende erste Gegenelement 208 des Operationsmikroskops hinein. Diese Position ist in Figur 4 dargestellt Diese Bewegung wird durch die dabei ansteigende magnetische Haltekraft unterstützt.

Figur 4 zeigt eine schematische Darstellung eines Schutzglasadapters gemäß Figur 3, der an einem Operationsmikroskop angeordnet ist.

Das Schutzglasmodul 210 gemäß Figur 4 weist die gleichen Komponenten auf wie das Schutzglasmodul 110 gemäß Figur 3.

An dieser Position ist die zweite Anlagefläche 206 des Operationsmikroskops 202 in Kontakt mit der ersten Anlagefläche 213 des Schutzglasadapters 211. Das erste Positionierungselement 216 ist in dem korrespondierenden ersten Gegenelement 208, einer Bohrung, angeordnet. Die Kontaktlinie zwischen dem Positionierungselement 216 und dem Gegenelement 208 kann eine Kreislinie bilden.

In dieser Position ist die maximale magnetische Haltekraft zwischen dem ersten Halteelement 218, einem ferromagnetischen Eisenelement, und dem ersten Gegen-Halteelement 228, einem Dauermagneten, ausgebildet. Es kann auch ein in Figur 3 und Figur 4 nicht dargestelltes zweites Halteelement am Schutzglasadapter 211 und ein korrespondierendes, nicht dargestelltes, viertes Halteelement am Operationsmikroskop 202 vorhanden sein. Damit ist eine noch stärkere Haltekraft zwischen Schutzglasadapter 211 und Operationsmikroskop 202 erreichbar.

Zum Lösen des Schutzglasadapters 211 von dem Operationsmikroskop 202 wird der Schutzglasadapter 211 leicht um die Zentralachse 114 gedreht. Dabei gleitet das erste Positionierungselement 216 aus dem korrespondierenden erste Gegenelement 208 heraus und der Abstand zwischen der ersten Anlagefläche 213 und er zweiten Anlagefläche 206 wird vergrößert. Das erste Halteelement 218 und das ersten Gegen-Halteelement 228 fluchten nicht mehr, und weisen durch die Drehung einen Versatz zueinander auf. In Kombination mit dem vergrößerten Abstand zwischen den Halteelementen verringert sich die magnetische Haltekraft fast auf null. Der Schutzglasadapter kann somit sehr einfach von dem Operationsmikroskop 202 wieder entfernt werden.

Die oben genannten Ausführungen gelten in gleicher Weise für einen Schutzglasadapter 211 mit oder ohne einem Objektivschutzglas 212.

### Bezugszeichenliste

- 1: Operationsmikroskop mit Schutzglasmodul
- 2: Operationsmikroskop
- 3, 203: Hauptobjektiv
- 4, 204: Optische Achse
- 5, 205: Äußere Mantelfläche
- 6, 206: Zweite Anlagefläche
- 7: Zweiter Kegelwinkel
- 8, 208: Erstes Gegenelement
- 10, 110, 210: Schutzglasmodul
- 11, 111, 211: Schutzglasadapter
- 12, 112, 212: Objektivschutzglas
- 13, 113, 213: Erste Anlagefläche
- 14, 114, 214: Zentralachse
- 15, 115, 215: Führungsöffnung
- 16, 116, 216: Erstes Positionierungselement
- 17: Erster Kegelwinkel
- 118, 218: Erstes Halteelement
- 119: Zweites Halteelement
- 120: Griff
- 121: Erstes Rastelement
- 122: Zweites Rastelement
- 123: Drittes Rastelement
- 124: Führungsschiene
- 125: Erster Doppelpfeil (Verschiebungsrichtung)
- 126: Zweites Positionierungselement
- 228: Drittes Halteelement
- 230: Abstand
- 231: Zweiter Doppelpfeil (Rotationsrichtung)
- 232: Dritter Doppelpfeil (Verschiebungsrichtung)
- 233: Pfeil (Verschieberichtung)

## Patentansprüche

1. Schutzglasadapter (11; 111; 211) für ein Operationsmikroskop (2; 202), aufweisend
- eine erste Anlagefläche (13; 113; 213),
wobei die erste Anlagefläche (13; 113; 213) derart ausgebildet ist, dass diese mit einer korrespondierenden zweiten Anlagefläche (6; 206) des Operationsmikroskops (2; 202) in Kontakt bringbar ist,
- eine Führungsöffnung (15, 115, 215) zur Aufnahme eines Hauptobjektivs (5, 205) des Operationsmikroskops (2, 202), wobei die Führungsöffnung (15, 115, 215) als Kegelstumpf mit einer ersten Zentralachse (14; 114; 214) ausgebildet ist, wobei der größere Durchmesser des Kegelstumpfes zu der ersten Anlagefläche (13; 113; 213) hin ausgebildet ist,
**gekennzeichnet durch**:
- mindestens ein Positionierungselement (16; 116; 216)
wobei das mindestens eine Positionierungselement (16; 116; 216) starr ausgebildet ist und an der ersten Anlagefläche (13; 113; 213) oder an der Oberfläche der Führungsöffnung (15; 115; 215) angeordnet ist,
wobei das Positionierungselement (16; 116; 216) zur formschlüssigen Aufnahme in einem Gegenelement (8; 208) an dem Operationsmikroskop (2; 202) geeignet ist.
- mindestens ein Halteelement (118; 218),
wobei durch das Halteelement (118; 218) eine Haltekraft parallel zu der ersten Zentralachse (14; 114; 214) zwischen dem Schutzglasadapter (11; 111; 211) und dem Operationsmikroskop (2; 202) bewirkbar ist, wobei die Haltekraft durch eine Magnetkraft gebildet ist.

2. Schutzglasadapter (11; 111; 211) nach Anspruch 1,
wobei das mindestens eine Positionierungselement (16; 116; 216) halbkugelförmig ausgebildet ist.

3. Schutzglasadapter (11; 111; 211) nach einem der vorherigen Ansprüche,
wobei zwei Positionierungselemente (16; 116, 126; 216) an der ersten Anlagefläche (13; 113; 213) oder an der Oberfläche der Führungsöffnung (15, 115, 215) angeordnet sind.

4. Schutzglasadapter (11; 111; 211) nach einem der vorherigen Ansprüche,
wobei drei Positionierungselemente an der ersten Anlagefläche (13; 113; 213) oder an der Oberfläche der Führungsöffnung (15, 115, 215) angeordnet sind.

5. Schutzglasadapter (11; 111; 211) nach einem der vorherigen Ansprüche,
aufweisend mindestens zwei Halteelemente (118, 119).

6. Schutzglasadapter (11; 111; 211) nach einem der vorherigen Ansprüche,
wobei die Führungsöffnung (15, 115, 215) einen Kegelwinkel zwischen 10° und 45°, bevorzugt zwischen 10° und 20°, aufweist.

7. Schutzglasadapter (11; 111; 211) nach einem der vorherigen Ansprüche,
wobei der Schutzglasadapter (11; 111; 211) ein Objektivschutzglas (12, 112, 212) umfasst.

8. Schutzglasadapter (11; 111; 211) nach Anspruch 7,
wobei das Objektivschutzglas (12, 112, 212) trennbar mit dem Schutzglasadapter (11: 111; 211) verbunden ist und in einer Aufnahmevorrichtung (124) aufgenommen ist.

9. Schutzglasadapter (11; 111; 211) nach einem der vorherigen Ansprüche,
wobei an dem Schutzglasadapter (11; 111; 211) eine Mikroskopabdeckung angeordnet ist.

10. Schutzglasadapter (11; 111; 211) nach Anspruch 9,
wobei die Mikroskopabdeckung an der ersten Anlagefläche (13; 113; 213) angeordnet ist.

11. Operationsmikroskop (2, 202) mit einem Schutzglasadapter (11; 111; 211) nach einem der vorherigen Ansprüche, wobei das Operationsmikroskop (2; 202) aufweist
- eine zweite Anlagefläche (6, 206), die mit der ersten Anlagefläche (13; 113; 213) des Schutzglasadapters (11; 111; 211) in Kontakt bringbar ist,
- ein Hauptobjektiv (3, 203) mit einer äußeren Umfangsfläche, wobei die äußere Umfangsfläche als Kegelstumpf ausgebildet ist, wobei der größere Durchmesser des Kegelstumpfes zu der zweiten Anlagefläche hin ausgebildet ist,
- mindestens ein Gegenelement (8, 208), das in Position und Form mit dem mindestens einen Positionierungselement (16, 116, 216) in Formschluss bringbar ist.
- mindestens ein Gegen-Halteelement (228), das derart ausgebildet ist, dass eine magnetische Haltekraft zwischen dem mindestens einen Halteelement (218) und dem mindestens einen Gegen-Halteelement (228) bewirkbar ist.

12. Operationsmikroskop (2, 202) mit einem Schutzglasadapter (11; 111; 211) nach Anspruch 11,
wobei die Führungsöffnung (15, 115, 215) des Schutzglasadapters (11; 111; 211) über das Hauptobjektiv (3, 203) geführt ist,
wobei der Schutzglasadapter (11; 111; 211) rotatorisch um die Zentralachse (14; 114; 214) beweglich ist, wenn das mindestens eine Positionierungselement (16; 116; 216) in Kontakt mit der zweiten Anlagefläche (6; 206) steht, sodass die erste Anlagefläche (13; 113; 213) durch das Positionierungselement (16; 116; 216) in einem Abstand zu der zweiten Anlagefläche (6; 206) des Operationsmikroskops (2, 202) angeordnet ist, wenn das Positionierungselement (16; 116; 216) nicht in Formschluss mit dem Gegenelement (8, 208) positioniert ist,
wobei die Haltekraft zwischen dem mindestens einen Halteelement (118; 218) und dem mindestens ein Gegen-Halteelement (228) ausschließlich an einer ersten Position ausgebildet ist, an der das Positionierungselement (16; 116; 216) an einer vorbestimmten Position in Bezug auf das Operationsmikroskop (2, 202) positioniert ist und das Positionierungselement (16; 116; 216) in dem Gegenelement(8, 208) in Bezug auf das Operationsmikroskop (2, 202) formschlüssig angeordnet ist, sodass die erste Anlagefläche (13; 113; 213) ausschließlich an der ersten Position mit der korrespondierenden zweiten Anlagefläche (6; 206) des Operationsmikroskops (2, 202) in Kontakt ist.

## Claims

1. Protection glass adapter (11; 111; 211) for a surgical microscope (2; 202), having
- a first abutment surface (13; 113; 213),
wherein the first abutment surface (13; 113; 213) is designed in such a way that it can be brought into contact with a corresponding second abutment surface (6; 206) of the surgical microscope (2; 202),
- a guide opening (15, 115, 215) for receiving a main objective (5, 205) of the surgical microscope (2, 202), wherein the guide opening (15, 115, 215) is designed as a truncated cone with a first central axis (14; 114; 214), wherein the greater diameter of the truncated cone is formed toward the first abutment surface (13; 113; 213),
**characterized by**:
- at least one positioning element (16; 116; 216), wherein the at least one positioning element (16; 116; 216) is rigid and is arranged on the first abutment surface (13; 113; 213) or on the surface of the guide opening (15; 115; 215),
wherein the positioning element (16; 116; 216) is suitable for being received with form-fit engagement in a mating element (8; 208) on the surgical microscope (2; 202),
- at least one holding element (118; 218),
wherein a holding force can be applied by the holding element (118; 218) parallel to the first central axis (14; 114; 214) between the protection glass adapter (11; 111; 211) and the surgical microscope (2; 202), wherein the holding force is formed by a magnetic force.

2. Protection glass adapter (11; 111; 211) according to Claim 1,
wherein the at least one positioning element (16; 116; 216)
has a hemispherical shape.

3. Protection glass adapter (11; 111; 211) according to either of the preceding claims,
wherein two positioning elements (16; 116, 126; 216) are arranged on the first abutment surface (13; 113; 213) or on the surface of the guide opening (15, 115, 215).

4. Protection glass adapter (11; 111; 211) according to one of the preceding claims,
wherein three positioning elements are arranged on the first abutment surface (13; 113; 213) or on the surface of the guide opening (15, 115, 215).

5. Protection glass adapter (11; 111; 211) according to one of the preceding claims,
having at least two holding elements (18, 119).

6. Protection glass adapter (11; 111; 211) according to one of the preceding claims,
wherein the guide opening (15, 115, 215) has a cone angle of between 10° and 45°, preferably between 10° and 20°.

7. Protection glass adapter (11; 111; 211) according to one of the preceding claims,
wherein the protection glass adapter (11; 111; 211) comprises an objective protection glass (12, 112, 212).

8. Protection glass adapter (11; 111; 211) according to Claim 7,
wherein the objective protection glass (12, 112, 212) is separably connected to the protection glass adapter (11; 111; 211) and is received in a receiving device (124).

9. Protection glass adapter (11; 111; 211) according to one of the preceding claims,
wherein a microscope drape is arranged on the protection glass adapter (11; 111; 211).

10. Protection glass adapter (11; 111; 211) according to Claim 9,
wherein the microscope drape is arranged on the first abutment surface (13; 113; 213).

11. Surgical microscope (2, 202) with a protection glass adapter (11; 111; 211) according to one of the preceding claims, wherein the surgical microscope (2; 202) has
- a second abutment surface (6, 206), which can be brought into contact with the first abutment surface (13; 113; 213) of the protection glass adapter (11; 111; 211),
- a main objective (3, 203) with an outer circumferential surface, wherein the outer circumferential surface is designed as a truncated cone, wherein the greater diameter of the truncated cone is formed toward the second abutment surface,
- at least one mating element (8, 208) which, in position and shape, can be brought into form-fit engagement with the at least one positioning element (16, 116, 216),
- at least one mating holding element (228), which is designed in such a way that a magnetic holding force can be effected between the at least one holding element (218) and the at least one mating holding element (228).

12. Surgical microscope (2, 202) with a protection glass adapter (11; 111; 211) according to Claim 11,
wherein the guide opening (15, 115, 215) of the protection glass adapter (11; 111; 211) is guided over the main objective (3, 203),
wherein the protection glass adapter (11; 111; 211) is movable in a rotary motion about the central axis (14; 114; 214) when the at least one positioning element (16; 116; 216)
is in contact with the second abutment surface (6; 206), such that the first abutment surface (13; 113; 213) is arranged by the positioning element (16; 116; 216) at a distance from the second abutment surface (6; 206) of the surgical microscope (2, 202) when the positioning element (16; 116; 216) is not positioned in form-fit engagement with the mating element (8, 208),
wherein the holding force between the at least one holding element (118; 218) and the at least one mating holding element (228) is formed exclusively at a first position at which the positioning element (16; 116; 216) is positioned at a predetermined position in relation to the surgical microscope (2, 202) and the positioning element (16; 116; 216) is arranged in form-fit engagement in the mating element (8, 208) in relation to the surgical microscope (2, 202), such that the first abutment surface (13;113; 213) is in contact with the corresponding second abutment surface (6; 206) of the surgical microscope (2, 202) exclusively at the first position.

## Revendications

1. Adaptateur de protection (11; 111; 211) pour microscope d'opération (2; 202), présentant une première surface de pose (13; 113; 213), la première surface de pose (13; 113; 213) étant configurée de manière à pouvoir être mise en contact avec une deuxième surface de pose (6; 206) correspondante du microscope d'opération (2; 202),
une ouverture de guidage (15, 115, 215) qui reprend un objectif principal (5, 205) du microscope d'opération (2, 202), l'ouverture de guidage (15, 115, 215) étant configurée sous la forme d'un tronc de cône présentant un premier axe central (14; 114; 214), le plus grand diamètre du tronc de cône étant formé du côté de la première surface de pose (13; 113; 213), **caractérisé par**
au moins un élément de positionnement (16; 116; 216), le ou les éléments de positionnement (16; 116; 216) étant rigides et disposés sur la première surface de pose (13; 113; 213) ou sur la surface de l'ouverture de guidage (15; 115; 215),
l'élément de positionnement (16; 116; 216) convenant pour être repris en correspondance géométrique dans un élément complémentaire (8; 208) prévu sur le microscope d'opération (2; 202),
au moins un élément de maintien (118; 218), une force de maintien parallèle au premier axe central (14; 114; 214) pouvant être appliquée par l'intermédiaire de l'élément de maintien (118; 218) entre l'adaptateur de protection (11; 111; 211) et le microscope d'opération (2; 202), la force de maintien étant une force magnétique.

2. Adaptateur de protection (11; 111; 211) selon la revendication 1, dans lequel le ou les éléments de positionnement (16; 116; 216) ont la forme d'une demi-sphère.

3. Adaptateur de protection (11; 111; 211) selon l'une des revendications précédentes, dans lequel deux éléments de positionnement (16; 116; 126; 216) sont disposés sur la première surface de pose (13; 113; 213) ou sur la surface de l'ouverture de guidage (15, 115, 215).

4. Adaptateur de protection (11; 111; 211) selon l'une des revendications précédentes, dans lequel trois éléments de positionnement sont disposés sur la première surface de pose (13; 113; 213) ou sur la surface de l'ouverture de guidage (15, 115, 215).

5. Adaptateur de protection (11; 111; 211) selon l'une des revendications précédentes, présentant au moins deux éléments de maintien (118, 119).

6. Adaptateur de protection (11; 111; 211) selon l'une des revendications précédentes, dans lequel l'ouverture de guidage (15, 115, 215) présente un angle de cône compris entre 10° et 45° et de préférence entre 10° et 20°.

7. Adaptateur de protection (11; 111; 211) selon l'une des revendications précédentes, dans lequel l'adaptateur de protection (11; 111; 211) comporte un verre de protection d'objectif (12, 112, 212).

8. Adaptateur de protection (11; 111; 211) selon la revendication 7, dans lequel le verre de protection d'objectif (12, 112, 212) est relié de manière amovible à l'adaptateur de protection (11; 111; 211) et est repris dans un ensemble de reprise (124).

9. Adaptateur de protection (11; 111; 211) selon l'une des revendications précédentes, dans lequel un recouvrement de microscope est disposé sur l'adaptateur de protection (11; 111; 211).

10. Adaptateur de protection (11; 111; 211) selon la revendication 9, dans lequel le recouvrement de microscope est disposé sur la première surface de pose (13; 113; 213).

11. Microscope d'opération (2, 202) doté d'un adaptateur de protection (11; 111; 211) selon l'une des revendications précédentes, le microscope d'opération (2; 202) présentant
une deuxième surface de pose (6, 206) qui peut être mise en contact avec la première surface de pose (13; 113; 213) de l'adaptateur de protection (11; 111; 211),
un objectif principal (3, 203) doté d'une surface périphérique extérieure, la surface périphérique extérieure étant configurée en forme de tronc de cône, le plus grand diamètre du tronc de cône étant formé du côté de la deuxième surface de pose,
au moins un élément complémentaire (8, 208) dont la position et la forme lui permettent d'être amené en correspondance géométrique avec le ou les éléments de positionnement (16, 116, 216) et
au moins un élément complémentaire de maintien (228) configuré de manière à pouvoir appliquer une force magnétique de maintien entre le ou les éléments de maintien (218) et le ou les éléments complémentaires de maintien (228).

12. Microscope d'opération (2, 202) présentant un adaptateur de protection (11; 111; 211) selon la revendication 11,
l'ouverture de guidage (15, 115, 215) de l'adaptateur de protection (11; 111; 211) pouvant être passée au-dessus de l'objectif principal (3, 203),
l'adaptateur de protection (11; 111; 211) pouvant se déplacer en rotation autour du premier axe central (14; 114; 214) lorsque le ou les éléments de positionnement (16; 116; 216) sont en contact avec la deuxième surface de pose (6; 206) de telle sorte que la première surface de pose (13; 113; 213) soit disposée par l'élément de positionnement (16; 116; 216) à une distance de la deuxième surface de pose (6; 206) du microscope d'opération (2, 202) lorsque l'élément de positionnement (16; 116; 216) n'est pas placé en correspondance géométrique avec l'élément complémentaire (8, 208),
la force de maintien entre le ou les éléments de maintien (118; 218) et le ou les éléments complémentaires de maintien (228) est formée uniquement en une première position sur laquelle l'élément de positionnement (16; 116; 216) est placé en une position prédéterminée par rapport au microscope d'opération (2, 202) et l'élément de positionnement (16; 116; 216) est disposé en correspondance géométrique par rapport au microscope d'opération (2, 202) dans l'élément complémentaire (8, 208) de telle sorte que la première surface de pose (13; 113; 213) soit en contact uniquement en la première position avec la deuxième surface de pose (6; 206) correspondante du microscope d'opération (2, 202).
